# EUROPEAN PATENT SPECIFICATION

(11) **EP 1 967 188 B1**
(45) Date of publication and mention of the grant of the patent: **25.01.2012**
(21) Application number: 06834556.0
(22) Date of filing: 13.12.2006
(51) Int. Cl.: A61K 31/282, A61J 1/00, A61K 9/08, A61K 47/04, A61P 35/00, B65D 81/24, B65D 85/82

(54) **PHARMACEUTICAL PREPARATION OF AN AQUEOUS SOLUTION CONTAINING PLATINUM COMPLEX**
PHARMAZEUTISCHE ZUBEREITUNG EINER WÄSSRIGEN LÖSUNG MIT EINEM PLATINKOMPLEX
PREPARATION PHARMACEUTIQUE D'UNE SOLUTION AQUEUSE CONTENANT UN COMPLEXE DE PLATINE

(30) Priority: 29.12.2005 JP 2005380617
(43) Date of publication of application: 10.09.2008
(73) Proprietor: NIPRO CORPORATION, Kita-ku, Osaka-shi, Osaka-fu, 531-8510 (JP)
(72) Inventor: NISHIDA, Seiji, Osaka-shi Osaka 531-8510 (JP); KATSUMATA, Takashi, Osaka-shi Osaka 531-8510 (JP); SATO, Makoto, Osaka-shi Osaka 531-8510 (JP)
(74) Representative: Albrecht, Thomas
(86) International application number: PCT/JP2006/324801
(87) International publication number: WO 2007/074641

(56) References cited:
- WO-A-2004/112778
- WO-A-2006/103691
- JP-A- 07 053 368
- JP-A- 11 124 329
- JP-A- 11 124 329

## Description

### Technical Field

The present invention relates to an aqueous pharmaceutical preparation containing a platinum complex. More specifically, the present invention relates to a stable aqueous pharmaceutical preparation in which an aqueous solution containing a platinum complex is contained in a gas-permeable container; and the gas-permeable container is contained in an oxygen-barrier container.

### Background Art

In recent years, malignant tumors tend to increase as a cause of death, so various kinds of anti-tumor agents have been developed. Platinum complexes know as anti-tumor agents include divalent platinum complexes such as cisplatin and carboplatin, and tetravalent platinum complexes such as iproplatin.
Cisplatin may cause a serious renal failure as a side effect. In addition, the administration route of cisplatin has been limited because cisplatin shows poor solubility in water and organic solvents. Carboplatin is a useful anti-tumor agent as the nephrotoxicity thereof is smaller than that of cisplatin.

At present, a commercially-available injectable carboplatin preparation is a freeze-dried powder preparation to be dissolved in use or a 1%-concentration aqueous pharmaceutical preparation using a small amount of a solution. Aqueous carboplatin preparations have been sold as pharmaceutical preparations in vial containers (see Non-Patent Documents 1 to 3).
Any of those preparations may be mixed with, for example, a physiological saline solution or a 5% glucose solution and then used in dilution before administration to a patient. In this case, the redissolving or diluting procedure should be sterile. Contamination of microorganisms or foreign matters in a drug solution during the preparation of the solution should be avoided from the viewpoint of preventing a cancer patient who may have lowered immunity from bacterial infection.
Also, a drug has cytotoxicity, so the exudation of the drug to the outside when the drug solution is sucked or discharged with a syringe must be avoided for the safety of a compounder. On this account, the preparation should be carried out with the greatest care, so a burden has been imposed on the compounder.

As measures for preventing compounders from drug exposure as described above and reducing burdens on the compounders, a method of enhancing the safety and simpleness of the prescription work using an aqueous pre-filled syringe pharmaceutical preparation has been proposed.
In general, the pre-filled syringe pharmaceutical preparation is convenient for the use in emergency and for the safety and simpleness in preparation. In this case, a syringe is filled with a drug solution so that the syringe is free of gas to the extent possible.

However, it has been known that the stability of an aqueous carboplatin solution can be improved in the presence of air (oxygen) in the container. According to Patent Document 1, for the stabilization of the aqueous carboplatin solution, when a liquid and an upper space in the container are purged with air or oxygen, it may be preferable that the liquid in the container account for 50% or less of the total volume of the container, or the volume of the upper space not filled with the liquid account for 50% or more of the total volume of the container. In fact, when the aqueous carboplatin solution accounts for 100% of a glass container in the absence of air (oxygen), sediment is undesirably precipitated in the aqueous carboplatin solution.
Therefore, when a glass-container syringe is used, there is a need for providing a sufficient space to prevent precipitation from occurring. Thus, the container is made in large size. For example, 50 mL of an aqueous carboplatin solution provided as a pharmaceutical preparation requires a 50-mL additional space in the container, so the container should have an inner volume of 100 mL or more.

Such a large-volume pre-filled syringe pharmaceutical preparation is difficult in handling because the inner diameter of a syringe barrel inevitably increases and an increased pressure for the injection of a drug solution is required.
In addition, there is a need for operation to remove air from the syringe barrel to mix the preparation with an infusion solution when the syringe barrel has a large inner space, so the operation can be complicated.
Further, in the case of a pharmaceutical preparation of a cytotoxic compound such as carboplatin, a drug solution may be dispersed outside (aerosolization phenomenon) by operation such as the removal of air from a syringe, through the amount of the dispersed solution is small. Accordingly, a compounder may be exposed to the drug.

In view of the foregoing, Patent Document 2 discloses a pre-filled syringe pharmaceutical preparation of an aqueous carboplatin solution using an oxygen-permeable syringe made of resin.
Patent Document 2 describes that the use of the oxygen-permeable syringe allows the aqueous carboplatin solution to be stable even without any space formed in the syringe and a compact pre-filled syringe pharmaceutical preparation can be thus produced.
However, actually the pharmaceutical preparation of Patent Document 2 has never been manufactured and the long storage stability of the preparation is not always adequately satisfied as an ethical drug.

Therefore, a compact pre-filled syringe pharmaceutical preparation containing a platinum complex stable even in long-term storage as an ethical drug has been desired in medical scenes.

Patent Document 1: JP-A-07-53368
Patent Document 2: JP-A-11-124329
Non-Patent Document 1: Attached document for ethical drug, "Paraplatin (registered trademark) injection solution", revised in September 2005.
Non-Patent Document 2: Attached document for ethical drug, "Carbomerk (registered trademark) injection solution", revised in September 2005.
Non-Patent Document 3: Attached document for ethical drug, "Carboplatin injection solution 1%-"hexal"", revised in September 2005.

### Disclosure of the Invention

### Problems to be solved by the Invention

The present invention intends to provide an aqueous pharmaceutical preparation of a platinum complex, which can be prepared by a simple operation, has a compact size, and suppresses the deterioration of a cyctotoxic drug even in prolonged storage without a fear of contamination of a compounder with the drug.

### Means for solving the Problems

The inventors of the present invention have completed the present invention by finding out means for solving the above problems as a result of intensive studies.
That is, the present invention relates to an aqueous pharmaceutical preparation of a platinum complex in a stable state, in which an aqueous solution containing a platinum complex is contained in an oxygen-permeable container, and the oxygen-permeable container is contained in an oxygen-barrier container, characterized in that a gap between the oxygen-barrier container and the oxygen-permeable container is filled with gas containing oxygen.
In addition, the present invention relates to a method of manufacturing an aqueous solution of a platinum complex, characterized by including: adding the platinum complex to a solvent while aerating the solvent with air or oxygen gas; and stirring the mixture to dissolve the platinum complex in the solvent.

### Effects of the Invention

The aqueous pharmaceutical preparation containing the platinum complex of present invention can be provided as a pre-filled syringe pharmaceutical preparation, a bag pharmaceutical preparation, or the like previously filled therewith, or can be a pharmaceutical preparation to be directly mixed in an infusion solution or a pharmaceutical preparation which can be directly injected into the vein. Therefore, for example, there is no need for the conventional operation for mixing a drug solution from a vial to an infusion bag, so the operation for preparation of the drug can be simplified and the burden on a compounder can be reduced. In addition, there is no need for using a syringe for suction, discharge, and so on, so the number of waste materials contaminated with an anticancer drug can be also reduced. Besides, the time required for preparation can be shortened and the number of preparation instruments and the like can be also reduced.

The conventional aqueous pharmaceutical preparation containing a platinum complex should be injected and mixed in an infusion container after a drug solution has been sucked from a vial, so the scattering or the like of the drug can occur. In contrast, the aqueous pharmaceutical preparation of the present invention does not require the operations of sucking and discharging the drug solution, and just requires direct injection into the infusion container, so it can be a safe preparation capable of reducing an opportunity of exposing a compounder to the drug. In the case of a bag pharmaceutical preparation containing a platinum complex at a concentration for use, there is no need for further injecting the preparation into an infusion container and thus the preparation can be directly used for a patient. In this way, a pharmaceutical preparation in good operable formulation is particularly significant for a drug having cytotoxicity.
In addition, a decrease in number of sucking and discharging the drug can reduce the opportunity of contaminating the drug at the time of preparation.

Further, the pharmaceutical preparation of the present invention has a gap between an oxygen-barrier container and an oxygen-permeable container filled with gas containing oxygen at high concentration. Therefore, a stable aqueous pharmaceutical preparation, in which the deterioration such as coloring of a drug solution is suppressed, is provided. The pharmaceutical preparation of the present invention is stable even without previously purging air (oxygen) into the container, and not to precipitate. Besides, there is no chance of the deposition of precipitation. In addition, the drug solution can be efficiently charged in the inner volume of the container, so the pharmaceutical preparation can be minimized.

### Best Mode for carrying out the Invention

Hereinafter, the present invention will be described in detail.
The platinum complex of the present invention is the collective designation of compounds each having coordinated divalent or tetravalent platinum. Specific examples of the platinum complex include carboplatin, cisplatin, oxaplatin, nedaplatin, and iproplatin. Of those, in particular, carboplatin is preferable as the platinum complex of the present invention.
The aqueous solution containing the platinum complex of the present invention is an aqueous solution containing any of the aforementioned platinum complexes. A solvent (water) to be used therein is generally water for injection. The content of the platinum complex in the aqueous solution is typically 0.01 to 20 mg/mL, preferably 0.5 to 18 mg/mL.
If the platinum complex is carboplatin, the concentration of carboplatin in the aqueous solution is preferably 1 to 18 mg/mL, particularly preferably 5 to 16 mg/mL. The pH of the aqueous solution is, but not specifically limited to, typically about 5 to 8, more preferably about 5 to 7.

For dissolving the platinum complex, agitation is preferably carried out while a solvent is being aerated with gas containing oxygen. In addition, the temperature of the solvent is kept at preferably 20 to 40°C, more preferably 25 to 35°C. The solvent is preferably water.

If required, the aqueous solution containing the platinum complex of the present invention may be added with a pH regulator, a tonicity adjusting agent, a stabilizing agent, and so on. In general, the aqueous solution contains neither a sulfur compound nor a divalent electrolytes such as calcium or magnesium. Further, it is preferable that the solution be free of the stabilizing agent such as sodium hydrogen sulfite.

The pH of the above aqueous solution is typically 2 to 8, preferably 5 to 8, more preferably 5 to 7. In the case of a bag pharmaceutical preparation containing a platinum complex at a concentration for use, it is preferable that the aqueous solution have a pH value of 6 to 7.5 and a titratable acidity of 10 or less to prevent a patient from causing vascular pain when the solution is intravenously administered to the patient.

In the present invention, the oxygen-permeable container is, but not specifically limited as far as the material thereof has good oxygen permeability, preferably made of resin. The resin may be any of various resin materials commonly used in medical containers and so on, and is preferably a polyalkylene-based resin or a polyvinyl-based resin. Specific examples of the resin preferably include polyalkylene-based resins such as polypropylene (PP), polymethyl pentene (TPX), and polyethylene (PE), and polyvinyl-based resins such as polyvinyl chloride (PVC) and polyvinyl acetate. In particular, PP, TPX, PE, and PVC are preferable.
In addition, the oxygen-permeable container of the present invention is preferably made of a material which is capable of bearing heat sterilization such as radiofrequency sterilization or high-pressure steam sterilization after production in consideration of hygiene and safety standpoints.

The oxygen permeability of the container used in the present invention is generally 100 cc/(m²·day·atm) or more, preferably 500 cc/(m²·day·atm) or more, more preferably 1,000 cc/(m²·day·atm) or more when it is determined by the method described below.
The oxygen permeability is determined on the basis of a differential pressure method, "Test method for gas transmission rate of plastic film and sheet" as described in the JIS standard (1987 Ed.) K7126 (page 826).

The oxygen-permeable container used in the present invention is generally in the form of a pre-filled syringe. However, it may be in the form of any of bags, vials, bottles, and so on as far as it attains the object of the present invention. For example, it can be also used in a bag pharmaceutical preparation storing an aqueous solution of a platinum complex at a low concentration for use.
In addition, the volume of the oxygen-permeable container is generally 1 to 1,500 mL, preferably 3 to 500 mL, particularly preferably 5 to 100 mL. The wall thickness of the container has only to be such that the container has an appropriate strength and good oxygen permeability, and is generally about 0.05 mm to 5 mm, more preferably about 0.1 mm to 2 mm, though the value varies depending on materials to be used.

The amount of an aqueous platinum complex solution to be contained in the above oxygen-permeable container is generally 50 to 100%, preferably 70 to 100%, more preferably 90 to 100% with respect to the inner volume of the oxygen-permeable container.

An aqueous platinum (II) compound solution can be charged into the oxygen-permeable container on the basis of the known method.
For example, there is a method in which an oxygen-permeable container is filled with the aqueous platinum (II) compound solution under an oxygen-containing gas atmosphere and then plugged, followed by heat sterilization, or there is a method in which it is aseptically filled under an oxygen-containing gas atmosphere and then plugged.

In the present invention, the oxygen-barrier container is substantially a container that does not allow oxygen to permeate in and out of the container. The oxygen permeability of the container is generally 5.0 cc/(m²·day·atm) or less, preferably 3.0 cc/(m²·day·atm) or less, more preferably 1. 0 cc/(m²·day·atm) or less.
Note that the oxygen permeability is determined on the basis of a differential pressure method, "Test method for gas transmission rate of plastic film and sheet" as described in the JIS standard (1987 Ed.) K7126 (page 826).

The above oxygen-barrier container is preferably made of resin. The resin may be any of various resin materials commonly used in medical containers and so on. Any of films, sheets, and so on made of various kinds of materials widely used for medical purposes can be generally used as a material of the container. Specific examples of the material include: ethylene/vinyl alcohol copolymers, polyvinylidene chloride, polyacrylonitrile, polyvinyl alcohol, polyamide, polyester, nylon, and the like; resin films each containing at least one of them; and laminated films on which these resins are coated or laminated.
A commercially-available material of the oxygen-barrier container is preferably, for example, a TECHBARRIER (registered trademark) manufactured by Mitsubishi Plastics, Inc.
In addition, the material may be a metalized film on which a metallic oxide such as silicon oxide or aluminum oxide is deposited from the vapor. A resin on which a metal is deposited from the vapor preferably, for example, nylon or polyethylene terephthalates.
In addition to the above resin-made container, the material of the oxygen-barrier container may be metallic foil made of a metal such as aluminum on which surface processing is carried out for preventing the container from reacting with a drug solution.
The oxygen-barrier container may be in the form of any of bags, bottles, and so on. In terms of storage, the container is preferably in the form of a flexible bag.

In the present invention, the gap between the oxygen-barrier container and the oxygen-permeable container is the external space portion of the oxygen-permeable container in the internal space of the oxygen-barrier container.
In the aqueous pharmaceutical preparation of the present invention, the gap is filled with gas containing oxygen.
The concentration of oxygen in the gas containing oxygen used in the present invention is generally 10 to 99% by mole, preferably 20 to 99% by mole, more preferably 30 to 99% by mole, still more preferably 40 to 99% by mole. The gas may contain another kind of gas except oxygen such as air or nitrogen; preferably the other kind of gas is nitrogen. To be specific, for example, the gas may be gas containing oxygen and nitrogen at a constant molar ratio of 2 : 3 or the like.
The inclusion of gas containing oxygen in the above gap can be carried out by a known method.

The pharmaceutical preparation of the present invention can be used as an anticancer drug and typically coadministrated with an infusion solution of 250 mL or more in volume per dose and with approximately 480 mg to 640 mg of carboplatin. A general bag pharmaceutical preparation may be prepared by premixing carboplatin with an infusion solution. In this case, however, an infusion solution with good blending stability should be chosen. Thus, for example, any of alcohol solutions of sugars such as mannitol, sorbitol, and xylitol as well as a glucose solution may be used. Alternatively, a bag pharmaceutical preparation having a plurality of chambers for separately storing carboplatin and an infusion solution may be prepared.

Hereinafter, the present invention will be described in more detail with reference to the following examples. However, the present invention will not be limited to these examples.

### Example 1

### <Preparation of aqueous platinum complex solution>

Carboplatin in amount of 9 g as a platinum (II) complex was added with water for injection at 30°C aerated with oxygen gas, stirred, and dissolved, followed by filling up to 900 mL.

### <Filtration sterilization and filling of oxygen-permeable container>

The above aqueous carboplatin solution was sterilized by filtration with a 0.22-µm membrane filter and 5 mL thereof was then aseptically charged in each pre-filled syringe container made of polypropylene (PP).

### <Outer packaging with oxygen-barrier container>

The oxygen-permeable container containing the aqueous carboplatin solution obtained as described above was contained in an oxygen-barrier bag (material: TECHBARRIER (registered trademark) manufactured by Mitsubishi Plastics, Inc., with an oxygen permeability of 1.0 cc/(m²·day·atm) or less). Subsequently, the opening of the oxygen-barrier bag was heat-sealed off in a state where the gap between the oxygen-permeable container and the oxygen-barrier bag was filled with gas containing 40% of oxygen and 60% of nitrogen. Consequently, the aqueous pharmaceutical preparation containing the platinum complex of the present invention was obtained.
Likewise, an aqueous pharmaceutical preparation was also obtained using the oxygen-barrier bag prepared from an aluminum-deposited sheet (i.e., a sheet made of nylon on which aluminum was deposited from the vapor).

### Example 2

An aqueous pharmaceutical composition containing a platinum complex was obtained in a manner similar to Example 1 except that the gas filling the gap between the oxygen-permeable container and the oxygen-barrier container was replaced with air containing 21% of oxygen.

### Example 3

An aqueous pharmaceutical composition containing a platinum complex was obtained in a manner similar to Example 1 except that the gas filling the gap between the oxygen-permeable container and the oxygen-barrier container was replaced with gas containing 10% of oxygen and 90% of nitrogen, air containing 21% of oxygen, gas containing 30% of oxygen and 70% of nitrogen, gas containing 40% of oxygen and 60% of nitrogen, or gas containing 50% of oxygen and 50% of nitrogen. Further, the oxygen-barrier container used was a TECHBARRIER (registered trademark, manufactured by Mitsubishi Plastics, Inc.) as in the case of Example 1.

### (Comparative Example 1)

An aqueous pharmaceutical composition was obtained by carrying out the processes of "preparation of aqueous platinum complex solution" and "filtration sterilization and filling of oxygen-permeable container" described in Example 1 but not carrying out "outer packaging with oxygen-barrier container" described in Example 1.

### (Comparative Example 2)

A vial pharmaceutical preparation was self-produced, which was similar to the already commercially-available Paraplatin (registered trademark, manufactured by Bristol-Myers K.K.).
However, unlike any of Examples 1 to 3 and Comparative Example 1, the aeration of oxygen gas was not carried out at the time of dissolving carboplatin.

### (Comparative Example 3)

An aqueous pharmaceutical composition containing a platinum complex was obtained in a manner similar to Example 3 except that the gas filling the gap between the oxygen-permeable container and the oxygen-barrier container was replaced with gas containing 0% of oxygen and made of high-purity nitrogen.

Hereinafter, test examples for the invention of the present application will be described.

### (Test Example 1) Test 1 for stability comparison

The pharmaceutical preparation obtained in Example 1, Example 2, Comparative Example 1, or Comparative Example 2 was subjected to 10-, 20-, or 30-day storage (storage conditions: 50°C, 75%RH) and its titer at each time point was then determined using a high-performance liquid chromatograph (HPLC) method.
The corresponding value of the measurement when the theoretical value was 100 was represented in Table 1.
A change in content of carboplatin with time is illustrated with respect to the pharmaceutical preparation obtained in Example 1, Example 2, Comparative Example 1, or Comparative Example 2.
The results are listed in Table 1.

The measurement was carried out by employing the following HPLC conditions for the quantitative assay of carboplatin.
Apparatus: liquid chromatograph manufactured by Hitachi, Ltd.
Column: COSMOSIL NH₂-MS (manufactured by Nacalai Tesque, Inc.)
Detection: 230 nm
Injection volume: 20 µL
Sample concentration: about 0.1 mg/mL
Temperature: constant temperature around 30°C
Mobile phase: acetonitrile/water mixture solution (4 : 1)
Flow rate: approximately 1 mL/min
Measurement time: 15 minutes

**Table 1**

| Sample | | | n | Storage period | | | |
|---|---|---|---|---|---|---|---|
| | Packaging | Packaging material | | Initial value | 10 days | 20 days | 30 days |
| Example 1 | Packaging after replacement with oxygen/nitrogen = 2:3 (Oxygen conc.: 40%) | TECHBARRIER | 1 | 97.5 | 95.5 | 96.0 | 94.0 |
| | | | 2 | 96.0 | 97.5 | 95.5 | 96.5 |
| | | | 3 | 97.0 | 98.5 | 96.0 | 98.0 |
| | | | Mean | 96.8 | 97.2 | 95.8 | 96.2 |
| | | Aluminum deposition | 1 | 97.5 | 96.0 | 97.0 | 95.0 |
| | | | 2 | 96.0 | 97.5 | 96.0 | 98.0 |
| | | | 3 | 97.0 | 98.5 | 97.5 | 97.0 |
| | | | Mean | 96.8 | 97.3 | 96.8 | 96.7 |
| Example 2 | Packing after replacement with air (Oxygen conc.: 21%) | TECHBARRIER | 1 | 97.5 | 95.5 | 96.5 | 94.5 |
| | | | 2 | 96.0 | 97.0 | 96.0 | 96.5 |
| | | | 3 | 97.0 | 98.0 | 98.5 | - |
| | | | Mean | 96.8 | 96.8 | 97.0 | 95.5 |
| | | Aluminum deposition | 1 | 97.5 | 96.0 | 97.5 | 92.5 |
| | | | 2 | 96.0 | 97.5 | 97.0 | 93.5 |
| | | | 3 | 97.0 | 99.0 | 97.0 | 96.5 |
| | | | Mean | 96.8 | 97.5 | 97.2 | 94.2 |
| Comparative Example 1 | Nonpackaging | - | 1 | 97.5 | 96.0 | 96.5 | 94.5 |
| | | | 2 | 96.5 | 97.5 | 96.0 | 96.5 |
| | | | 3 | 97.0 | 97.0 | 97.0 | 98.0 |
| | | | Mean | 96.8 | 96.8 | 96.5 | 96.3 |
| Comparative Example 2 | Vial pharmaceutical preparation (space portion: air) | | 1 | 98.5 | 93.5 | 95.5 | 94.5 |
| | | | 2 | 97.0 | 94.5 | 95.0 | 93.5 |
| | | | 3 | 97.5 | 96.0 | 96.0 | 94.5 |
| | | | Mean | 97.7 | 94.7 | 95.5 | 94.2 |
| Titer (%) | | | | | | | |

### (Test Example 2)

Pharmaceutical preparations obtained in Example 1, Example 2, Comparative Example 1, and Comparative Example 2 were each stored for 10-, 20-, and 30-days (storage conditions: 50°C and 75%RH) and their transmissivities (T₄₃₀%) were then determined.
Water was used as a reference and its transmissivity at 430 nm was then determined using a spectrophotometer. The measurement values were listed in Table 2.
Apparatus: Hitachi spectrophotometer

**Table 2**

| Sample | | | n | Storage period | | | |
|---|---|---|---|---|---|---|---|
| | Packaging | Packaging material | | Initial value | 10 days | 20 days | 30 days |
| Example 1 | Packaging after replacement with oxygen/nitrogen = 2:3 (Oxygen conc.: 40%) | TECHBARRIER | 1 | 98.3 | 96.7 | 93.8 | 93.1 |
| | | | 2 | 98.2 | 96.2 | 93.5 | 93.5 |
| | | | 3 | 98.1 | 96.5 | 92.9 | 92.9 |
| | | | Mean | 98.2 | 96.5 | 93.4 | 93.2 |
| | | Aluminum deposition | 1 | 98.3 | 97.4 | 93.2 | 92.7 |
| | | | 2 | 98.2 | 97.0 | 93.4 | 92.7 |
| | | | 3 | 98.1 | 97.0 | 93.5 | 91.8 |
| | | | Mean | 98.2 | 97.1 | 93.4 | 92.4 |
| Example 2 | Packing after replacement with air (Oxygen conc.: 21%) | TECHBARRIER | 1 | 98.3 | 94.8 | 88.8 | 85.0 |
| | | | 2 | 98.2 | 94.6 | 89.0 | 86.8 |
| | | | 3 | 98.1 | 93.7 | 89.2 | 86.1 |
| | | | Mean | 98.2 | 94.4 | 89.0 | 86.0 |
| | | Aluminum deposition | 1 | 98.3 | 94.9 | 88.9 | 85.8 |
| | | | 2 | 98.2 | 94.0 | 87.8 | 86.9 |
| | | | 3 | 98.1 | 94.2 | 89.7 | 86.0 |
| | | | Mean | 98.2 | 94.4 | 88.8 | 86.2 |
| Comparative Example 1 | Nonpackaging | - | 1 | 98.3 | 94.0 | 87.9 | 85.7 |
| | | | 2 | 98.2 | 93.2 | 87.7 | 86.3 |
| | | | 3 | 98.1 | 93.8 | 87.8 | 86.9 |
| | | | Mean | 98.2 | 93.7 | 87.8 | 86.1 |
| Comparative Example 2 | Vial pharmaceutical preparation (space portion: air) | | 1 | 98.2 | 95.8 | 91.5 | 91.0 |
| | | | 2 | 98.0 | 94.4 | 91.0 | 89.0 |
| | | | 3 | 98.0 | 94.6 | 88.8 | 89.1 |
| | | | Mean | 98.1 | 94.9 | 90.4 | 89.7 |
| Transmissivity (T₄₃₀%) | | | | | | | |

### (Test Example 3)

The pharmaceutical preparation obtained in Example 3, Comparative Example 2, or Comparative Example 3 was subjected to 10-, 20-, or 30-day storage (storage conditions: 50°C, 75%RH) and its titer at each time point was then determined using the HPLC method in the same manner as in Test Example 1.
The results are listed in Table 3.

**Table 3**

| Sample | n | Storage period (Day) | | | |
|---|---|---|---|---|---|
| Packaging | | Beginning | 10 | 20 | 30 |
| Packaging after replacement with oxygen/nitrogen = 0:10 : Comparative Example 3 (Oxygen conc.: 0%) | 1 | 100.8 | 98.0 | 91.7 | 95.2 |
| | 2 | 102.3 | 99.2 | 93.6 | 90.6 |
| | 3 | 101.7 | 100.2 | 87.6 | 93.6 |
| | Mean | 101.6 | 99.1 | 91.0 | 93.1 |
| Packaging after replacement with oxygen/nitrogen = 1:9 (Oxygen conc.: 10%) | 1 | 101.5 | 98.3 | 97.4 | 97.4 |
| | 2 | 102.5 | 99.2 | 99.0 | 99.3 |
| | 3 | 101.4 | 101.0 | 100.3 | 97.7 |
| | Mean | 101.8 | 99.5 | 98.9 | 98.1 |
| Packaging after replacement with air (Oxygen conc.: 21%) | 1 | 101.6 | 98.5 | 98.0 | 97.1 |
| | 2 | 102.9 | 99.3 | 99.7 | 98.7 |
| | 3 | 101.2 | 100.3 | 99.7 | 97.7 |
| | Mean | 101.9 | 99.4 | 99.1 | 97.8 |
| Packaging after replacement with oxygen/nitrogen = 3:7 (Oxygen conc.: 30%) | 1 | 101.2 | 98.8 | 97.4 | 97.9 |
| | 2 | 102.4 | 99.1 | 98.9 | 99.2 |
| | 3 | 101.3 | 101.0 | 99.7 | 97.5 |
| | Mean | 101.6 | 99.7 | 98.7 | 98.2 |
| Packaging after replacement with oxygen/nitrogen = 2:3 (Oxygen conc.: 40%) | 1 | 101.6 | 99.6 | 98.0 | 97.7 |
| | 2 | 102.0 | 99.5 | 99.7 | 98.7 |
| | 3 | 100.3 | 100.5 | 99.7 | 97.4 |
| | Mean | 101.3 | 99.9 | 99.1 | 97.9 |
| Packaging after replacement with oxygen/nitrogen = 5:5 (Oxygen conc.: 50%) | 1 | 100.2 | 98.6 | 97.7 | 96.7 |
| | 2 | 101.2 | 98.8 | 98.9 | 99.1 |
| | 3 | 100.4 | 100.9 | 99.8 | 96.9 |
| | Mean | 100.6 | 99.4 | 98.8 | 97.6 |
| Vial pharmaceutical preparation: Comparative Example 2 (space portion: air) | 1 | 99.4 | 97.5 | 96.7 | 95.4 |
| | 2 | 99.8 | 98.7 | 98.6 | 97.4 |
| | 3 | 100.2 | 100.6 | 99.0 | 96.1 |
| | Mean | 99.8 | 98.9 | 98.1 | 96.3 |
| Titer (%) | | | | | |

### (Test Example 4)

Pharmaceutical preparations obtained in Example 3, Comparative Example 2, and Comparative Example 3 were each stored for 10-, 20-, and 30-days (storage conditions: 50°C and 75%RH) and their transmissivities (T₄₃₀%) were then determined in the same manner as in Test Example 2.
The results are listed in Table 4.

**Table 4**

| Sample | n | Storage period (Day) | | | |
|---|---|---|---|---|---|
| Packaging | | Beginning | 10 | 20 | 30 |
| Packaging after replacement with oxygen/nitrogen = 0:10 : Comparative Example 3 (Oxygen conc.: 0%) | 1 | 100.1 | 63.5 | 59.3 | 39.0 |
| | 2 | 100.2 | 60.3 | 59.5 | 39.1 |
| | 3 | 100.2 | 69.4 | 57.4 | 35.9 |
| | Mean | 100.2 | 64.4 | 58.7 | 38.0 |
| Packaging after replacement with oxygen/nitrogen = 1:9 (Oxygen conc.: 10%) | 1 | 100.1 | 93.9 | 85.9 | 78.4 |
| | 2 | 100.0 | 93.3 | 86.0 | 82.1 |
| | 3 | 100.2 | 93.7 | 87.0 | 82.4 |
| | Mean | 100.1 | 93.6 | 86.3 | 81.0 |
| Packaging after replacement with air (Oxygen conc.: 21%) | 1 | 100.2 | 96.8 | 88.6 | 88.2 |
| | 2 | 100.1 | 96.7 | 87.1 | 88.1 |
| | 3 | 100.2 | 96.8 | 88.8 | 87.8 |
| | Mean | 100.2 | 96.8 | 88.2 | 88.0 |
| Packaging after replacement with oxygen/nitrogen = 3:7 (Oxygen conc.: 30%) | 1 | 100.1 | 98.8 | 94.9 | 91.9 |
| | 2 | 100.3 | 99.0 | 94.2 | 92.1 |
| | 3 | 100.2 | 98.8 | 95.1 | 92.1 |
| | Mean | 100.2 | 98.9 | 94.7 | 92.0 |
| Packaging after replacement with oxygen/nitrogen = 2:3 (Oxygen conc.: 40%) | 1 | 100.1 | 99.2 | 96.4 | 94.4 |
| | 2 | 100.3 | 99.3 | 96.3 | 94.9 |
| | 3 | 100.0 | 99.2 | 96.4 | 94.6 |
| | Mean | 100.1 | 99.2 | 96.4 | 94.6 |
| Packaging after replacement with oxygen/nitrogen = 5:5 (Oxygen conc.: 50%) | 1 | 100.2 | 99.2 | 96.8 | 94.5 |
| | 2 | 100.2 | 99.5 | 96.9 | 94.5 |
| | 3 | 100.1 | 99.5 | 97.2 | 95.1 |
| | Mean | 100.2 | 99.4 | 97.0 | 94.7 |
| Vial pharmaceutical preparation: Comparative Example 2 (space portion: air) | 1 | 100.2 | 99.1 | 96.3 | 93.4 |
| | 2 | 100.0 | 99.1 | 96.3 | 94.5 |
| | 3 | 100.2 | 99.2 | 96.5 | 94.3 |
| | Mean | 100.1 | 99.1 | 96.4 | 94.1 |
| Transmissivity (T₄₃₀%) | | | | | |

As is evident from the results of the measurement for the titers in the respective drug solutions in Table 1, the pharmaceutical preparations of the respective examples each have almost the same stability as that of each of the comparative examples. However, in the results of the measurement for the coloring of the drug solutions, the coloring-suppressing effects of Examples 1 and 2 were comparable to or higher than those of Comparative Examples 1 and 2 particularly after a lapse of 30 days or more. In particular, it is found that the coloring of the drug solution in Example 1 can be apparently suppressed compared with the comparative examples.
Further, in the results shown in Table 3 or Table 4 obtained by the measurement of the titer or coloring of the drug at each oxygen concentration in Example 3, a small decrease in titer at an oxygen concentration of 10 to 50% and a coloring-suppressing effect comparable to or higher than that of Comparative Example 2 at an oxygen concentration of 40 or 50% were observed. In contrast, at an oxygen concentration of 0%, a significant increase in titer of the drug and the significant coloring of the drug were observed with Comparative Example 3.
As is evident from the above results, in the aqueous pharmaceutical preparation of the present invention, it is found that the presence of the oxygen-containing gas in the gap between the oxygen-barrier outer packaging and the oxygen-permeable inner container prevents the drug solution from deteriorating and the stable aqueous pharmaceutical preparation containing a platinum complex can be obtained.

### Industrial Applicability

The present invention is an aqueous pharmaceutical preparation containing a platinum complex with improved stability and can be advantageously used as, for example, an anti-tumor agent in medical sites. In addition, an aqueous pharmaceutical preparation in the form of a pre-filled syringe can be used after being directly mixed with an intravenous glucose infusion or a physiological saline solution.

## Claims

1. An aqueous pharmaceutical preparation of a platinum complex in a stable state, comprising an aqueous solution containing the platinum complex contained in an oxygen-permeable container, wherein the oxygen-permeable container is contained in an oxygen-barrier container, **characterized in that** a gap between the oxygen-barrier container and the oxygen-permeable container is filled with gas containing oxygen, wherein an oxygen concentration of the gas containing the oxygen is 30% to 99% by mole.

2. The aqueous pharmaceutical preparation according to claim 1, wherein the platinum complex comprises carboplatin.

3. The aqueous pharmaceutical preparation according to claim 1, wherein the oxygen-permeable container is made of a resin.

4. The aqueous pharmaceutical preparation according to claim 1, wherein the resin comprises a polyalkylene-based resin or a polyvinyl-based resin.

5. The aqueous pharmaceutical preparation according to claim 1, wherein the oxygen-permeable container comprises a pre-filled syringe, a plastic bottle, or a plastic bag.

6. The aqueous pharmaceutical preparation according to claim 1, wherein a wall of the oxygen-barrier container has an oxygen permeability of 5.0 cc/(m²·day·atm) or less.

7. The aqueous pharmaceutical preparation according to claim 1, wherein a content of the platinum complex in the aqueous solution containing the platinum complex is 0.01 to 20 mg/mL.

## Patentansprüche

1. Wässrige pharmazeutische Zubereitung eines Platinkomplexes in einem stabilen Zustand, die eine wässrige Lösung, die den Platinkomplex enthält, in einem für Sauerstoff permeablen Behälter enthalten umfasst, wobei der für Sauerstoff permeable Behälter in einem Sauerstoffbarriere-Behälter enthalten ist, **dadurch gekennzeichnet, dass** ein Spalt zwischen dem Sauerstoffbarriere-Behälter und dem für Sauerstoff permeablen Behälter mit einem Sauerstoff enthaltenden Gas gefüllt ist, wobei die Sauerstoffkonzentration des Sauerstoff enthaltenden Gases 30 bis 99 Mol-% beträgt.

2. Wässrige pharmazeutische Zubereitung gemäß Anspruch 1, wobei der Platinkomplex Carboplatin umfasst.

3. Wässrige pharmazeutische Zubereitung gemäß Anspruch 1, wobei der für Sauerstoff permeable Behälter aus einem Harz besteht.

4. Wässrige pharmazeutische Zubereitung gemäß Anspruch 1, wobei das Harz ein Polyalkylen-basiertes Harz oder ein Polyvinyl-basiertes Harz umfasst.

5. Wässrige pharmazeutische Zubereitung gemäß Anspruch 1, wobei der für Sauerstoff permeable Behälter eine vorgefüllte Spritze, eine Kunststoffflasche oder einen Kunststoffbeutel umfasst.

6. Wässrige pharmazeutische Zubereitung gemäß Anspruch 1, wobei eine Wand des Sauerstoffbarriere-Behälters eine Sauerstoffpermeabilität von 5, 0 cm³/ (m² · Tag · atm) oder weniger hat.

7. Wässrige pharmazeutische Zubereitung gemäß Anspruch 1, wobei die Gehalt des Platinkomplexes in der wässrigen Lösung, die den Platinkomplex enthält, 0,01 bis 20 mg/ml ist.

## Revendications

1. Préparation pharmaceutique aqueuse d'un complexe de platine en état stable qui comprend une solution aqueuse contenant le complexe de platine et contenue dans un récipient perméable à l'oxygène, dans laquelle le récipient perméable à l'oxygène est contenu dans un récipient de barrière à l'oxygène, **caractérisée en ce que** l'espace entre le récipient de barrière à l'oxygène et le récipient perméable à l'oxygène est rempli avec un gaz contenant de l'oxygène, la concentration de l'oxygène étant de 30 à 99% en mole.

2. Préparation pharmaceutique aqueuse selon la revendication 1, dans laquelle le complexe de platine comprend du carboplatine.

3. Préparation pharmaceutique aqueuse selon la revendication 1, dans laquelle le récipient perméable à l'oxygène est fabriqué d'une résine.

4. Préparation pharmaceutique aqueuse selon la revendication 1, dans laquelle la résine comprend une résine à base de polyalkylène ou une résine à base polyvinylique.

5. Préparation pharmaceutique aqueuse selon la revendication 1, dans laquelle le récipient perméable à l'oxygène comprend une seringue préremplie, une bouteille en plastique ou un sac en plastique.

6. Préparation pharmaceutique aqueuse selon la revendication 1, dans laquelle un paroi du récipient de barrière à l'oxygène a une perméabilité à oxygène inférieure ou égale à 5,0 cm³ / (m²·jour·atm).

7. Préparation pharmaceutique aqueuse selon la revendication 1, dans laquelle la teneur de la solution aqueuse contenant le complexe de platine est 0,01 à 20 mg/ml.
